Europäisches Patentamt

European Patent Office

Office européen des brevets

(19)

(11) Veröffentlichungsnummer: **0 154 334**
**B1**

(12) **EUROPÄISCHE PATENTSCHRIFT**

(45) Veröffentlichungstag der Patentschrift:
**10.01.90**

(21) Anmeldenummer: **85102516.3**

(22) Anmeldetag: **06.03.85**

(51) Int. Cl.⁴: **B 01 J 8/20, B 01 J 8/22,
B 01 J 19/24 // C02F3/28,
C12M1/04, C12P7/06**

(54) **Verfahren zur Durchführung von Reaktionen und Stoffaustauschprozessen in heterogenen fluiden Systemen.**

(30) Priorität: **08.03.84 DE 3408464**

(43) Veröffentlichungstag der Anmeldung:
**11.09.85 Patentblatt 85/37**

(45) Bekanntmachung des Hinweises auf die Patenterteilung:
**10.01.90 Patentblatt 90/2**

(84) Benannte Vertragsstaaten:
**AT CH DE FR GB IT LI NL**

(56) Entgegenhaltungen:
**EP-A- 0 089 486
DE-A- 2 830 126
US-A- 3 826 739**

(73) Patentinhaber: **VLT Gesellschaft für
verfahrenstechnische Entwicklung mbH, Augsburger
Strasse 708, D-7000 Stuttgart 61 (DE)**

(72) Erfinder: **Reule, Waldemar, Dr.-Ing., Neulingstrasse 16,
D-7121 Walheim (DE)**
Erfinder: **Schreiber, Georg, Dipl.-Ing., Kiefernweg 7,
D-5000 Köln 50 (DE)**

ACTORUM AG

**Beschreibung**

Die Erfindung bezieht sich auf ein Verfahren und Vorrichtung zur kontinuierlichen Durchführung von Reaktionen und Stoffaustauschprozessen in heterogenen fluiden Systemen, die suspendierte Feststoffe enthalten (fest-flüssig, fest-flüssig-gasförmig), unter Verwendung eines Reaktors mit Rührkesselcharakteristik, verbunden mit einem nachgeschalteten Reaktor mit Rohrcharakteristik.

Es ist bekannt, daß bei chemischen und biochemischen Reaktionen und bei Stoffaustauschprozessen wie Adsorption, Ionenaustausch, Absorption, Extraktion, Kristallisation, Rektifikation, Filtration usw. die Verweilzeitverteilung auf die Apparate- und Anlagengröße, auf Umsatz, Ausbeute und Selektivität sowie auf den Betriebsmittel- und Energieverbrauch einen wesentlichen Einfluß ausübt. Es ist auch bekannt, daß sich in einem Reaktionsapparat ohne axiale Rückvermischung, in einem sog. idealen Rohrreaktor, der sich durch Pfropfenströmung auszeichnet, bei einfachen isothermen Reaktionen kontinuierlich ein höherer Umsatz erreichen läßt als in einem durchmischten Reaktor gleichen Volumens, beispielsweise einem Rührkesselreaktor, wobei anstelle eines Rohrreaktors häufig mit dem gleichen Erfolg auch eine aus mehreren hintereinandergeschalteten Rührapparaten bestehende Kesselkaskade eingesetzt werden kann.

In bestimmten Fällen kann es sich auch als notwendig erweisen, einen Apparat mit Rührkesselcharakteristik als erster Stufe mit einem Apparat mit Rohrcharakteristik als zweiter Stufe zu kombinieren.

Durch die DE-OS 2 830 126 ist ein derartiges Verfahren und Vorrichtung zur Durchführung (bio-) chemischer Reaktion in fluiden Systemen bekannt geworden.

Nachteilig ist es bei diesem bekannten Verfahren, eine Komponente, z.B. eine Reaktionskomponente oder einen Hilfsstoff wie etwa suspendierte Katalysatorpartikeln nach der Reaktions- bzw. Austauschstufe von der Reaktionslösung abzutrennen und wieder in den Prozeß zurückzuführen. Dies erfordert einen großen Aufwand, insbesondere wenn diese Trennung zwecks Vermeidung hoher Energieverluste unter Druck und bei hohen Temperaturen durchgeführt werden muß. Eine Rückführung von Feststoffen, z.B. von Katalysatorpartikeln läßt sich dann nur durch zusätzliche aufwendige Fördereinrichtungen bewerkstelligen.

Aufgabe der Erfindung ist es daher, ein Verfahren zu schaffen, das ermöglicht, die geschilderten Nachteile zu vermeiden und eine Vorrichtung vorzuschlagen, um das Verfahren durchzuführen.

Diese Aufgabe wird dadurch gelöst, daß man das untere Ende des Reaktors mit Rohrcharakteristik mit dem unteren Ende des Reaktors mit Rührkesselcharakteristik verbindet und den Produktstrom aus dem Reaktor mit Rohrcharakteristik abnimmt und nur einen Teilstrom, der die festen Komponenten des Reaktionsgemisches (eingedickte Katalysatorsuspension) enthält, in den Reaktor mit Rührkesselcharakteristik zurückführt.

Weitere Gestaltungen der Erfindung sind in den Patentansprüchen 2 bis 13 aufgeführt.

Dadurch, daß die beiden Stufen durch Rohrleitungen miteinander verbunden werden, stellen sie ein einziges hydraulisches, kommunizierendes System dar. In die erste Stufe wird in bekannter Weise zum Zwecke der Durchmischung Energie eingetragen, beispielsweise hydromechanisch mittels eines Rührers, hydrostatisch durch Begasen oder hydrodynamisch durch einen Flüssigkeitstreibstrahl, was sich in Bewegungsenergie des Fluids im ersten Reaktor äußert und in diesem über die Bewegung des Fluids in Form von Strömungen zu einem gegenüber dem statischen Zustand veränderten Druckfeld führt. Es bilden sich dadurch Teilräume mit höherem und solche mit geringerem statischen Druck aus. Im ersten Reaktor, also in der ersten Stufe strömt dadurch Fluid von den Räumen höheren Druckes zu den Räumen niederen Druckes und infolge der Impulsübertragung durch das Rührorgan, also beispielsweise Rührer, Gasblasen oder Treibstrahl, unter Druckaufbau wieder zu den Räumen höheren Druckes.

Wird nun ein Raum höheren Druckes beispielsweise durch eine Rohrleitung außerhalb des ersten Reaktors mit einem Raum niederen Druckes verbunden, so strömt Fluid in diesem Druckgradienten durch die Verbindungsleitung, also parallel zu den Rückströmungen im ersten Reaktor. Dies nutzt die Erfindung aus, indem sie in die Verbindung zwischen den Räumen hohen und niederen Druckes im ersten Reaktor einen zweiten Reaktor einschaltet, der zum Zwecke der Reaktion bzw. des Stoffaustausches Rohrcharakteristik aufweist. Dadurch wird erreicht, daß ein Teilstrom des fluiden mehrphasigen Systems, der im ersten Reaktor unter den Bedingungen mehr oder weniger vollständiger Durchmischung an Reaktionen bzw. am Stoffaustausch teilgenommen hat, allein infolge der Impulswirkung im ersten Reaktor, also ohne zusätzliche Zufuhr weiterer externer Energie, unter Bedingungen von Pfropfenströmung durch die zweite Stufe fließt und unter dem Einfluß derselben Kräfte wieder in die erste Stufe zurückfließt, wobei in ihm die bezweckte weitgehende Umsetzung bzw. der Stoffaustausch stattfindet.

Die Tendenz des Teilstroms, ohne weitere Zufuhr von Energie, also z.B. ohne eine Pumpe, allein durch die Druckdifferenz zwischen den Räumen hohen und niederen Druckes durch die zweite Stufe hindurch und in die erste Stufe zurückzufließen, wird verstärkt, wenn in einem heterogenen Mehrphasensystem vor der Stelle, an welcher der Teilstrom des Fluids aus der ersten Stufe über die obere Verbindung in die zweite Stufe austritt, eine Phasentrennung auftritt derart, daß das Fluid (z.B. eine Gas-Flüssig-Dispersion oder eine Suspension) in diesem Teilstrom, also auch in der zweiten Stufe, eine höhere mittlere Dichte besitzt als das Mehrphasensystem in der ersten Stufe bzw. in den Rückströmungen von Räumen höheren zu Räumen niedrigeren Druckes innerhalb der ersten Stufe.

Ein solcher Fall liegt z.B. vor, wenn die erste Stufe ein Blasensäulenreaktor oder ein Mammut-Schlaufenreaktor ist, in welchem ein Gas die Mischenergie liefert und in welchem die Bewegungsenergie liefernde Gasphase (disperse Phase) vor Eintritt des Fluidums in die obere Verbindung aus dem Mehrphasensystem am oberen Ende der ersten Stufe austritt. In diesem Fall besitzt dann der Teilstrom durch die zweite Stufe eine höhere mittlere Dichte als das Gemisch im Blasensäulenreaktor oder im nach unten strömenden Teil des Umlaufstromes im Schlaufenreaktor.

Der Zulauf, im folgenden als Einsatzstrom bezeichnet, kann der ersten oder auch der zweiten Stufe zugeführt werden, wogegen der Ablauf, im folgenden als Produktstrom bezeichnet, aus der zweiten Stufe abgetrennt wird, und zwar an einer Stelle, an welcher der gewünschte Umsatz erreicht ist.

Die Erfindung wird anhand der Beschreibung und der Zeichnungen näher erläutert.

Es zeigen:

Figur 1 in schematischer Darstellung eine Ausführungsform der zur Durchführung des erfindungsgemäßen Verfahrens bestimmten zweistufigen Apparatur,

Figur 2 in vereinfachter schematischer Darstellung eine Variante, bei welcher der Einsatzstrom der zweiten Stufe zugeführt wird,

Figur 3 eine weitere Variante, bei welcher der Produktstrom in einer besonderen Trennvorrichtung abgeführt wird,

Figur 4 eine Ausführungsform mit inneren Umlauf in der als Mammutschlaufenreaktor ausgebildeten ersten Stufe,

Figur 5 eine Ausführungsform mit äußerem Umlauf in der als Mammutschlaufenreaktor ausgebildeten ersten Stufe,

Figur 6 die Integration der zweiten Stufe in die erste Stufe,

Figur 7 und 8 zwei Ausführungsformen mit als Wirbelzellenkolonne ausgebildeter zweiter Stufe,

Figur 9 ein Ausführungsbeispiel mit zwei Reaktoren als zweiter Stufe,

Figur 10 ein Ausführungsbeispiel mit Hintereinanderschaltung zweier ersten und zweiten Stufen,

Figur 11 ein Schema einer Anlage zum Betreiben des erfindungsgemäßen Verfahrens am Beispiel einer anaeroben Aufarbeitung von hochbelasteten Prozeßabwässern;

Figur 12 ein weiteres Schema einer Anlage zum Betreiben des erfindungsgemäßen Verfahrens und

Figur 13 die Anwendung der Erfindung in Form eines Blockschemas am Beispiel der Aufarbeitung eines säurehaltigen Abwassers.

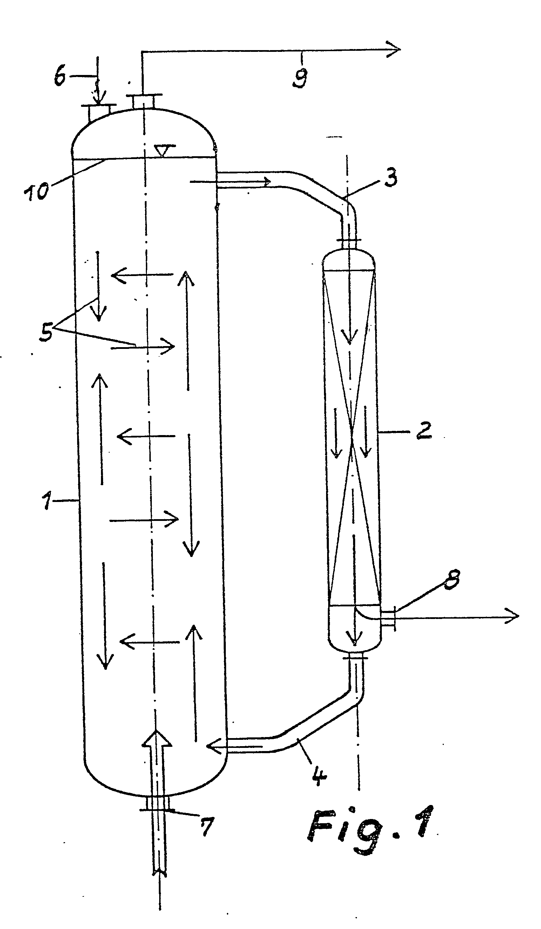

Nach Figur 1 sind die Reaktoren 1 der ersten Stufe und 2 der zweiten Stufe im Bereich ihrer oberen Enden mittels der Rohrleitung 3 und im Bereich ihrer unteren Enden mittels der Rohrleitung 4 miteinander verbunden. Wie durch die Pfeile 5 angedeutet, handelt es sich bei dem Reaktor 1 um einen durchmischten Apparat mit Rührkesselcharakteristik, beim Reaktor 2 dagegen um einen solchen mit Pfropfenströmung bzw. Rohrcharakteristik. Der Einsatzstrom wird durch den Eintrittsstutzen 6 eingetragen und die Mischenergie, beispielsweise Gas, durch den Stutzen 7. In Fällen, bei denen sich während der Reaktion Gas bildet, kann selbstverständlich dieses selbst nach dem bekannten Prinzip der Mammutpumpe als Antrieb für den Durchmischungsvorgang benutzt werden. Der Produktstrom wird am Austritt 8 abgenommen und das Reaktionsgas durch den Stutzen 9 abgeführt. Das Niveau des Fluidgemisches ist mit 10 bezeichnet.

Das durch den Stutzen 6 eingetragene Fluidgemisch wird durch die über Stutzen 7 eingetragene Mischenergie wie durch die Pfeile 5 symbolisch angedeutet, in Durchmischung gebracht. Die beiden Reaktorstufen 1 und 2 sind so angeordnet und ausgelegt, daß ein Teilstrom des durchmischten Fluidgemisches aus dem Reaktor 1 über die Leitung 3 in den Reaktor 2 fließt und diesen infolge seiner Ausbildung mit Rohrcharakteristik gezwungenermaßen in Form einer Pfropfenströmung durchfließt. Während an dem Austrittsstutzen 8, an welchem der gewünschte Umsatz erreicht ist, der Produktstrom abgenommen wird, fließen die rezirkulierten Komponenten über die Leitung 4 zurück in den Reaktor 1 und nehmen am weiteren Verfahrensablauf teil.

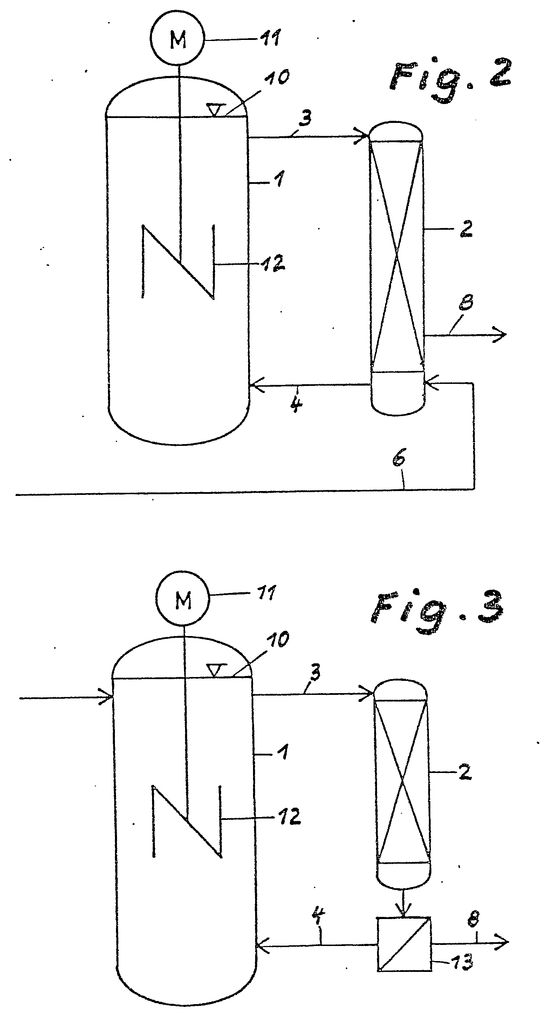

Wie aus Figur 2, bei der gleiche Teile wieder mit den gleichen Bezugszeichen versehen sind, ersichtlich, kann die Trennung in der zweiten Stufe beispielsweise durch den Austausch der das Produkt enthaltenden fluiden Phase gegen eine den Einsatz enthaltende andere fluide Phase durchgeführt werden. Die Durchmischung in der ersten Stufe, d.h. im Reaktor 1 erfolgt hier mittels des Rührers 12 durch den Mischantrieb 11.

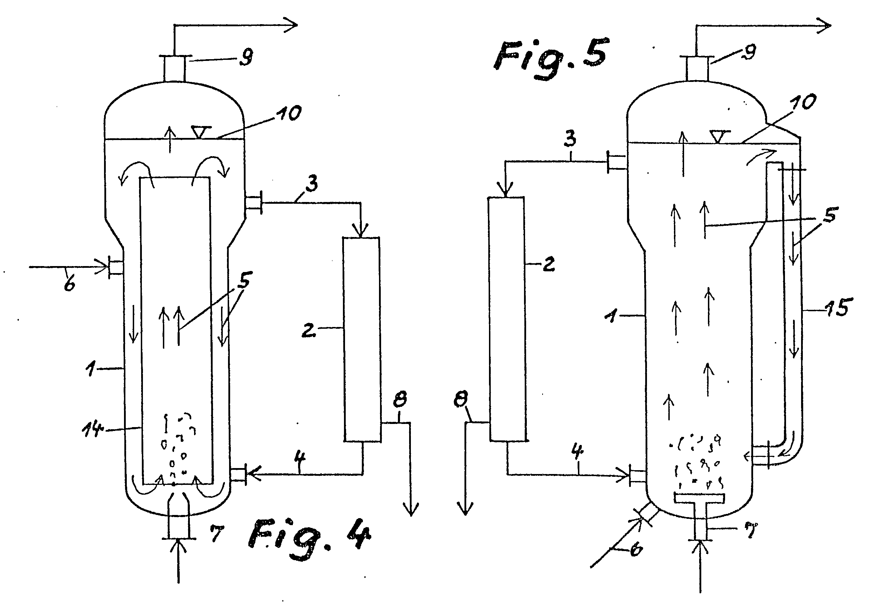

Wie Figur 3 zeigt, kann die Produktabnahme auch in einer in der Leitung 4, also außerhalb des Reaktors 2 angeordneten separaten Trennvorrichtung 13, die mit den Antrittsstutzen 8' versehen ist, beispielsweise durch Sedimentation, Filtration oder dgl. vorgenommen werden.

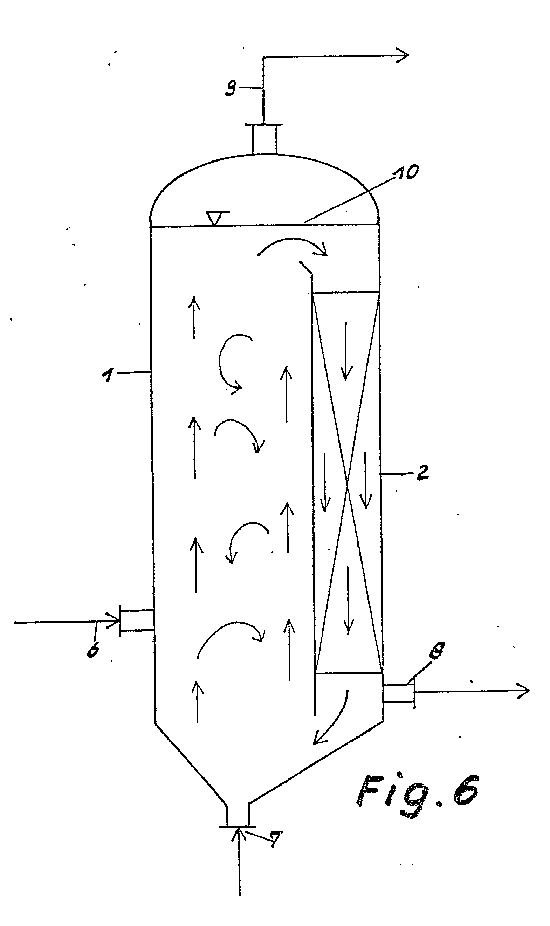

Der als Mammutschlaufenreaktor ausgebildete Reaktor 1 der ersten Stufe ist nach Figur 4 als zylindrischer Reaktor mit koaxial eingebautem Leitrohr 14 für inneren und nach Figur 5 als Blasensäule mit äußerer Rückführleitung 15 für äußeren Umlauf gemäß den Pfeilen 5 ausgebildet. Dadurch entstehen jeweils zwei Rohrbereiche bzw. Teilräume, die in Form einer geschlossenen Schlaufe durchströmt werden. Die Zufuhr von Gas, in der Regel in den größeren der beiden Teilräume, bewirkt den Umlauf der Flüssigkeit bzw. Suspension, die Schlaufenströmung. Die in der kontinuierlichen Phase dispergierten Gasblasen steigen in der Flüssigkeit (Suspension) infolge ihres Auftriebes (potentielle Energie) auf. Dabei wird Impuls in Form von Reibungs- und Schleppkräften auf das umgebende Fluid übertragen, das somit in der Richtung des Gases strömt. An der Oberfläche des Gemisches treten Gasblasen aus dem Fluid aus, das Fluid strömt dagegen mit einem geringeren Gehalt an Gasblasen (oder blasenfrei, je nach Möglichkeit des Ausgasens für die Blasen) im anderen Teilraum zurück nach unten.

Diese Vorgänge der Impulsübertragung zwischen Gasblasen und Fluid und das Austreten eines Gasstromes aus dem Fluid im stationären Zustand sind charakterisiert durch eine Differenz des Gasgehaltes zwischen den beiden Teilräumen, die auch existiert, wenn im Fall eines Reaktors, in dem durch die Reaktion Gas entsteht, die externe Gaszufuhr abgestellt wird. Im Teilraum mit dem größeren Volumen bleibt die nach oben gerichtete Strömung erhalten, weil dort mehr Gas entsteht als im kleineren, nach unten durchströmten Teilraum. Das entstehende Gas tritt an der Gemischoberfläche aus.

Die Gasgehaltsdifferenz zwischen den beiden Teilräumen ist gleichbedeutend mit einer Differenz der statischen Drücke zwischen den beiden Räumen (gemessen in gleicher Höhe). Dadurch resultiert also eine Schlaufenströmung entsprechend dem Druckfeld.

Diese Druckdifferenz wird erfindungsgemäß dazu benutzt, einen Teilstrom der Flüssigkeit (Suspension) aus der ersten Stufe durch die zweite Stufe der Vorrichtung ohne weitere Zufuhr von Energie zu fördern.

Bei der Ausführungsform der Figur 6 ist der Reaktor 2 der zweiten Stufe in den Reaktor 1 der ersten Stufe integriert, so daß hier die Leitungen 3 und 4 entfallen. Es versteht sich, daß die Produktentnahme aber auch hier im Bereich des integrierten Reaktors 2 an geeigneter Stelle erfolgen muß.

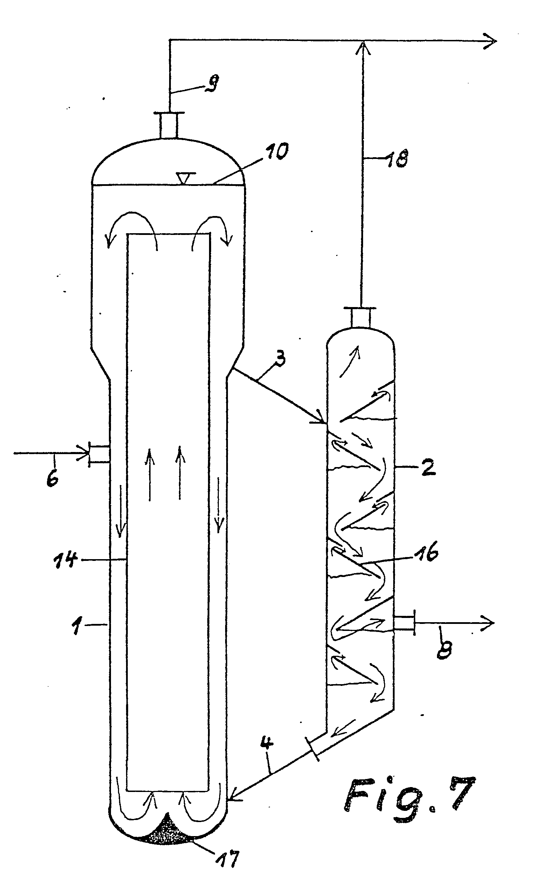

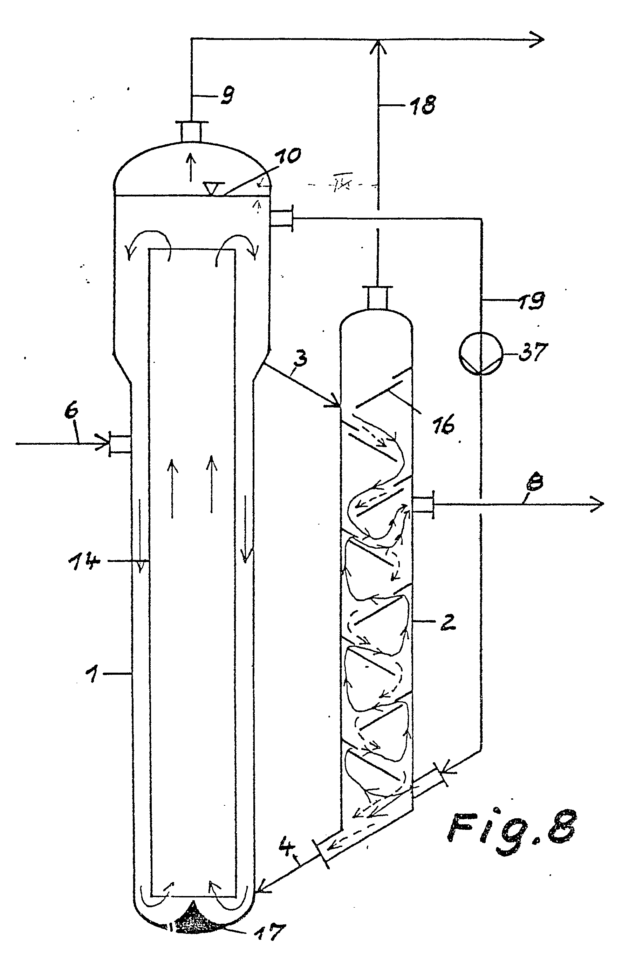

Nach Figur 7 ist der Reaktor 1 wiederum als Mammutschlaufenreaktor mit innerem Umlauf und der Reaktor 2 als eine Wirbelzellenkolonne mit den Schrägböden 16 ausgebildet. Der Reaktor 1 ist hier als Reaktor im Zusammenhang mit einer Reaktion mit Gasbildung vorgesehen, d.h. auf den Eintrag externer Mischenergie ist hier verzichtet. Zwecks Begünstigung der Strömung im Reaktor 2 ist der Reaktorboden 17 als Umlenkboden ausgebildet, was vor allem bei Suspensionen wichtig ist. Ferner ist am oberen Ende des Reaktors 2 zwecks Abführung von Reaktionsgas die Leitung 18 vorgesehen. Gemäß Figur 8 ist in einer Variante des weiteren noch die Leitung 19 mit Pumpe 37 vorgesehen, über welche dem Reaktor 1 an geeigneter Stelle ein Teilstrom entnommen und dem Reaktor 2 unten zum Zwecke des Austausches der kontinuierlichen Phase bzw. Rückführung suspendierter Partikeln in den Reaktor 1 zugeführt wird. Vorausgesetzt, daß an der Eintrittsstelle der Leitung 19 in den Reaktor 2 zwischen Leitung 19 und Reaktor 2 ein Druckgefälle vorhanden ist, kann auf die Verwendung der Pumpe 37 auch verzichtet werden.

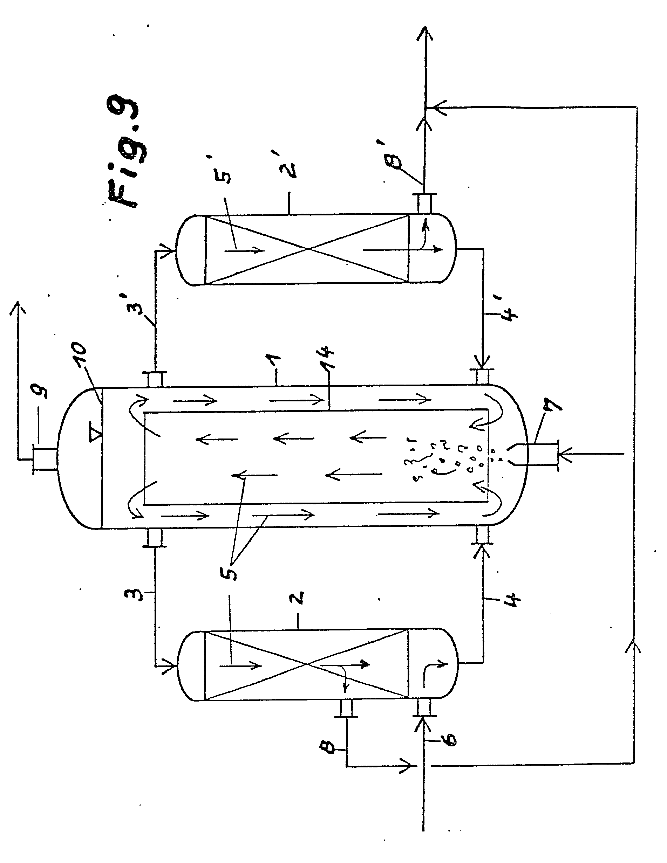

Das Ausführungsbeispiel der Figur 9 zeigt in Verbindung mit einem Reaktor 1 der ersten Stufe zwei Reaktoren 2 und 2' der zweiten Stufe. Der Einsatzstrom wird dabei dem einen Reaktor 2 der zweiten Stufe zugeführt, was den Vorteil hat, daß der Einsatzstrom die im unteren Teil dieses Reaktors sich befindenden Fluidgemischteile in den Reaktor 1 weitertransportiert. Selbstverständlich könnte der Einsatzstrom aber auch hier unmittelbar in den Reaktor 1 zugeführt werden.

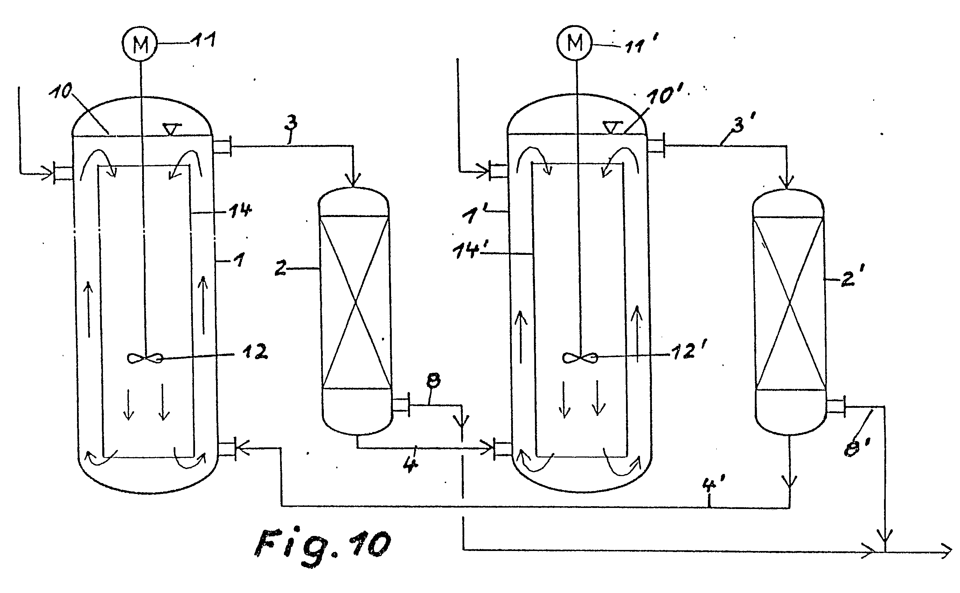

Nach Figur 10 sind zwei Reaktoren 1 und 1' der ersten Stufe vorgesehen, denen je ein Reaktor 2 und 2' der zweiten Stufe zugeordnet ist. Dabei ist vorgesehen, daß der Teilstrom des Reaktors 2 in den Reaktor 1' und der Teilstrom 2' in den Reaktor 1 fließt. Abweichend von der gezeigten Darstellung könnte auch vorgesehen sein, daß der Einsatzstrom nur dem Reaktor 1 zugeführt und der Produktstrom nur dem Reaktor 2' entnommen wird.

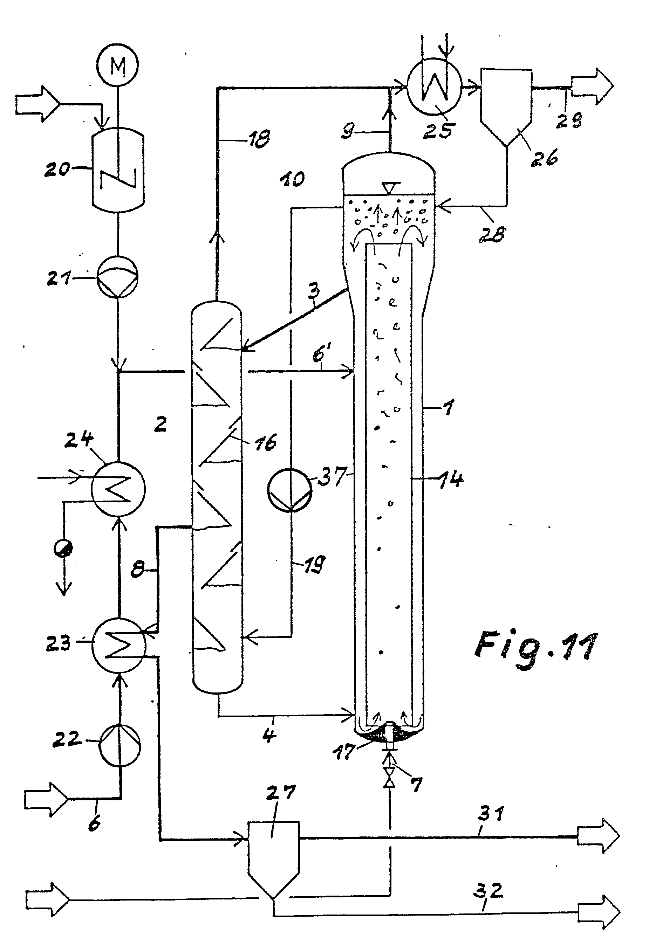

Die Figur 11 zeigt für das Beispiel einer anaeroben biologsichen Abwasserreinigung als erste Stufe den Mammutschlaufenreaktor, d.h. Reaktor 1 mit Leitrohr 14 und als zweite Stufe die Wirbelzellenkolonne, d.h. Reaktor 2 mit Schrägeboden 16, wobei beide im Bereich ihrer oberen Enden mittels der Leitung 3 und im Bereich ihrer unteren Enden mittels der Leitung 4 miteinander verbunden sind. Ferner sind der Reaktor 1 und 2 mittels der Leitung 19 miteinander verbunden. Die Abwasserzufuhr durch den Stutzen 6 in den Reaktor 1 erfolgt mittels der Abwasserpumpe 22 über den Abwasservorwärmer 23, der gleichzeitig als Reinwasser-Kühler dient, sowie über den dampfbeheizten Abwasserwärmetauscher 24. Aus dem Nährlösungsbehälter 20 wird über die Dosierpumpe 21 gleichzeitig die Nährlösung durch den Eintrittsstutzen 6 in den Reaktor 1 eingetragen. Die Zufuhr der Mischenergie, beispielsweise eines Gases wie Stickstoff, erfolgt durch den Stutzen 7. Anstelle dessen könnte aber auch vorgesehen sein, daß Biogas aus dem Kreislauf nach Verdichtung zum Antrieb verwendet wird. Die Entnahme des Produktstromes durch den Stutzen 8 aus dem Reaktor 2 erfolgt über den gleichzeitig als Abwasservorwärmer dienenden Reinwasserkühler 23 und den Reinwasser-Feststoffabscheider 27, wobei dann über die Leitung 31 das Reinwasser und über die Leitung 32 der Überschußschlamm erhalten wird. Das Reaktionsgas wird aus dem Reaktor 1 über die Leitung 9 und aus dem Reaktor 2 über die Leitung 18 ab- und dem Gaskühler 25 sowie dem Kondensatabscheider 26 zugeführt, wonach das Biogas über die Leitung 29 abgeführt und über die Leitung 28 das Kondensat in den Reaktor 1 zurückgeführt wird.

Betriebsbedingt können auch Fälle auftreten, bei denen das Mehrphasensystem in der zweiten Stufe eine geringere mittlere Dichte aufweist, als das Gemisch in der ersten Stufe. Die Differenz der mittleren Dichten des Mehrphasensystems in den beiden Stufen kann sogar so groß sein, daß die dadurch entstehende Differenz der hydrostatischen Drücke zwischen den beiden Stufen nicht mehr durch die in der ersten Stufe durch Impulsübertragung induzierte Druckdifferenz zwischen den Räumen hohen und niederen Drucks kompensiert werden kann.

Dieser Fall kann z.B. dann auftreten, wenn in einem solchen zweistufigen Reaktorsystem eine Suspension durch die beiden Stufen zirkulieren soll. Insbesondere beim Anfahren des Reaktorsystems, wenn u.U. die Partikel nur in die erste Stufe eingefüllt werden, aber auch während des stationären Betriebs, bedingt durch gewisse lokale Instabilitäten der Suspensionsströmung, kann die mittlere Dichte des Mehrphasensystems in der zweiten Stufe kleiner sein bzw. werden als in der ersten Stufe. Dann würde der Suspensionsstrom aus der ersten Stufe durch die zweite Stufe und zurück in die erste Stufe nicht in Gang bzw. zum Erliegen kommen.

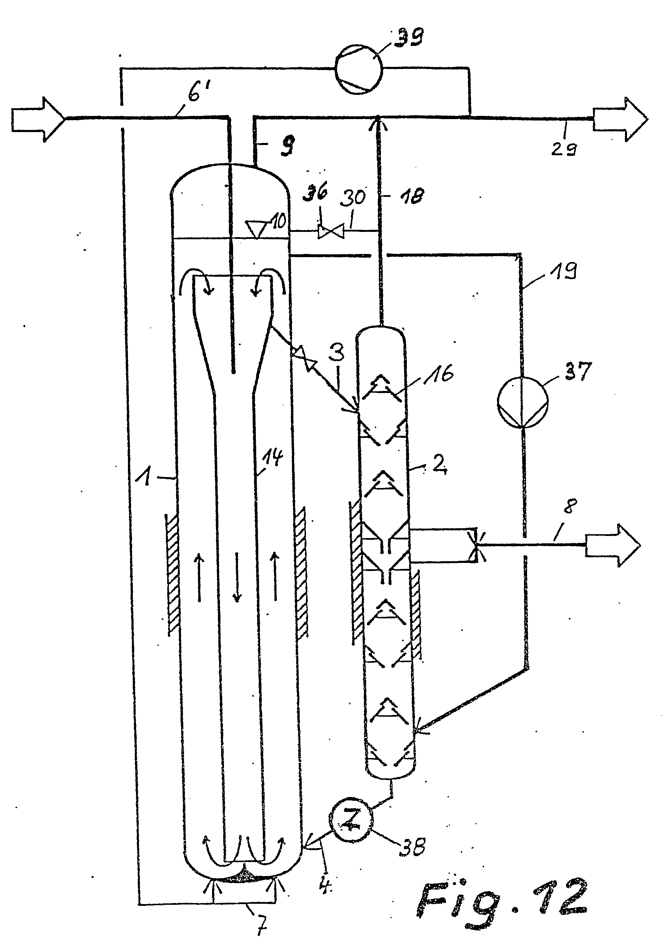

Nun zeigt sich aber in einem solchen Fall (Figur 12), wo neben der flüssigen, kontinuierlichen Phase noch eine schwerere, disperse Phase vorliegt, also bei einer Suspenion von Partikeln, die eine größere Dichte aufweisen als die Flüssigkeit, daß auch in dem Raum der zweiten Stufe, der oberhalb der Anschlußstelle der Leitung 3 in die zweite Stufe liegt, eine Entmischung der Suspension auftritt.

Sind nun die beiden Stufen, wie in den Figuren 7, 8 und 11 gezeigt, über weitere Leitungen 9 und 18 miteinander verbunden, die in die erste Stufe oberhalb des Niveaus des Flüssigkeitsgemisches 10 münden, so bildet sich auch in der zweiten Stufe bzw. in der Leitung 18 ein Niveau des Flüssigkeitsgemisches aus, dessen Niveau jedoch oberhalb des Niveaus des Flüssigkeitsgemisches 10 liegt. Diese Lage ist dadurch bedingt, daß in dem Raum der zweiten Stufe oberhalb der Einleitstelle des Teilstromes 3 infolge der Sedimentation der schweren dispersen Phase von der Einleitstelle nach unten nur die kontinuierliche flüssige Phase vorliegt, während in dem Raum der ersten Stufe oberhalb der Stelle, an welcher die Rohrleitung 3 von der ersten Stufe anschließt, ein Gemisch aus kontinuierlicher Phase und schwerer disperser Phase vorhanden ist. Da die entsprechenden Räume der beiden Stufen miteinander hydraulisch kommunizieren, muß in dem Raum mit der kleineren Fluiddichte das Flüssigkeitsniveau höher liegen als in dem Raum mit der höheren Dichte. Dieser Niveauunterschied ist auch dann vorhanden, wenn die mittlere Dichte der Suspension in der zweiten Stufe größer ist als in der ersten Stufe. Sie ist aber besonders groß, wenn die zweite Stufe nur mit der leichteren Flüssigkeit gefüllt ist.

Wird nun der Raum oberhalb des Niveaus des Flüssigkeitsgemisches 10 in der ersten Stufe über eine Leitung 30 mit dem Raum der zweiten Stufe verbunden, die oberhalb der Entmischungszone, also oberhalb der Anschlußstelle der Rohrleitung 3 in der zweiten Stufe liegt, so fließt die leichtere Flüssigkeit über diese Leitung 30 unter der Wirkung des hydrostatischen Drucks der Flüssigkeitssäule, welche oberhalb des Niveaus des Flüssigkeitsgemisches 10 in der ersten Stufe liegt, in die erste Stufe zurück. Dadurch sinkt der hydrostatische Druck an der Einleitstelle des Teilstroms 3 in die zweite Stufe, wodurch mehr Suspension aus der ersten durch die Leitung 3 in die zweite Stufe strömt. Der Teilstrom der Flüssigkeit, die durch die Leitung 30 strömt, wird durch die Druckdifferenz, die zwischen den Anfangs- und Endpunkten der Leitung 3 infolge der Dichtedifferenz der Suspensions- bzw. Flüssigkeitssäule erhalten bleibt, aufrechterhalten. Durch die Trennung des Flüssigkeitsstromes durch die Leitung 30 vom Suspensionsstrom durch die Leitung 3 am oberen Ende der zweiten Stufe wird die Suspension an dieser Stelle eingedickt, so daß die Konzentration der Suspension an schwerer Phase in der zweiten Stufe beim Anfahren immer größer wird bzw. im stationären Betrieb immer größer ist als in der ersten Stufe. Damit wird bzw. ist auch die mittlere Dichte in der zweiten Stufe immer größer als in der ersten Stufe und damit ein sicherer Betrieb hinsichtlich des umlaufenden Teilstroms durch die zweite Stufe gewährleistet. Durch die Drosselung des Flüssigkeitsstroms in die Leitung 30, z. B. mit einem Ventil 36, ist es möglich, den Suspensionsstrom in der Leitung 3 und damit die Konzentration der schwereren dispersen Phase in der zweiten Stufe einzustellen.

In Figur 12 ist auch der Fall gezeigt, wo das Biogas mittels des Verdichters 39 verdichtet und zum Antrieb des Umlaufs im Reaktor 1 (Mammutschlaufenreaktor) benutzt wird und eine Drossel- bzw. Zuteilungseinrichtung zwischen den Reaktoren mit Rührkesselcharakteristik und Rohrcharakteristik angeordnet ist.

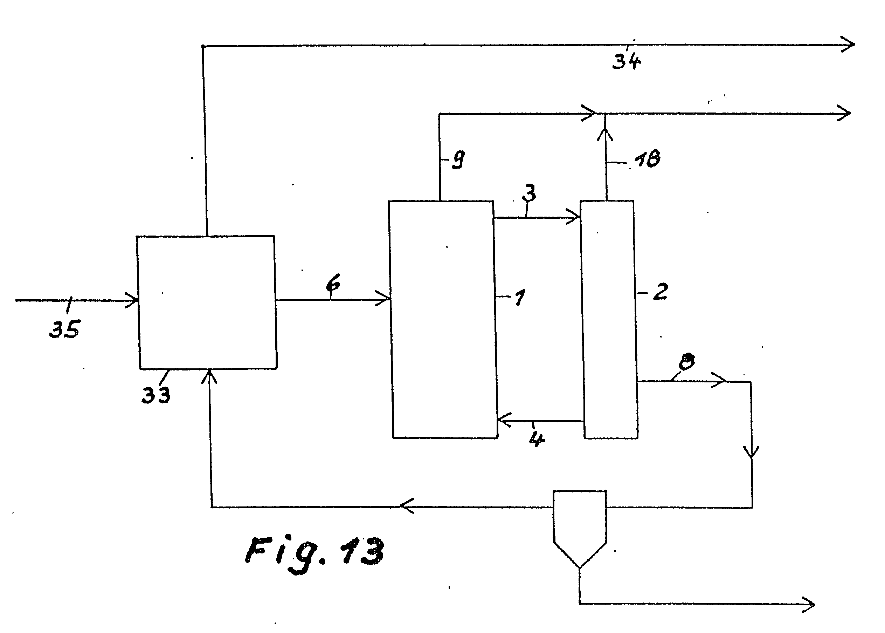

Bei dem Schema für die Aufbereitung säurehaltigen Prozeßwassers gemäß Figur 13 wird das hier Essigsäure enthaltende Abwasser durch die Leitung 35 zunächst dem Ionenaustauscher 33 zugeführt, von wo ein Teil des Abwassers mit $CO_2$ als gereinigtes Wasser über die Leitung 34 abgeleitet wird, ein anderer Teil des Abwassers mit CHCOONa dem Reaktor 1 zufließt, der wieder mittels der Leitungen 3 und 4 mit dem Reaktor 2 verbunden ist. Während am oberen Ende des Reaktors 1 und dem Reaktor 2 über die Leitungen 9 bzw. 18 als Produkt $CH_4 + CO_2$ abgeführt werden, wird der Reaktor 2 an geeigneter Stelle bei 8 Abwasser mit $NaHCO_3$ entnommen und dem Ionenaustauscher 33 zugeführt, so daß Natriumionen aus dem Abwasser entfernt und in die Reaktion zurückgeführt werden.

Nach dem erfindungsgemäßen Verfahren lassen sich besonders vorteilhaft
 – Solid-Liquid-Systeme mit mindestens einer festen Phase, dispergiert in mindestens einer flüssigen, kontinuierlichen Phase sowie
 – Gas-Liquid-Systeme mit mindestens einer Gasphase und mindestens einer festen Phase, dispergiert in mindestens einer flüssigen, kontinuierlichen Phase
betreiben. Dabei kommen für die erste Stufe
 – Rührkessel als Schlaufenreaktoren mit Propellerrührer
 – Schlaufenreaktoren mit Antrieb des Umlaufs durch Mammutpumpe
 – Schlaufenreaktoren mit Antrieb des Umlaufs durch Flüssigkeitsstrahl (Strahl-Schlaufen-Reaktor)
 – Blasensäulen
 – Rührkessel
und für die zweite Stufe
 – Wirbelzellenkolonnen oder ähnliche vielstufige Kaskadenkolonnen mit selbstfluidisierenden Zellen (Stufen)

– Kolonnen mit Einbauten aus statischen Mischern

  – Siebboden-Kolonnen

  – Füllkörper-Kolonnen

  – gepackte Kolonnen

in Betracht.

## Patentansprüche

1. Verfahren zur Durchführung von Reaktionen und Stoffaustauschprozessen in heterogenen fluiden Systemen, die suspendierte Feststoffe enthalten (fest-flüssig, fest-flüssig-gasförmig), unter Verwendung eines Reaktors mit Rührkesselcharakteristik, verbunden mit einem nachgeschalteten Reaktor mit Rohrcharakteristik, dadurch gekennzeichnet, daß man das untere Ende des Reaktors mit Rohrcharakteristik (2) mit dem unteren Ende des Reaktors mit Rührkesselcharakteristik (1) verbindet und den Produktstrom aus dem Reaktor mit Rohrcharakteristik (2) abnimmt und nur einen Teilstrom, der die festen Komponenten des Reaktionsgemisches (eingedickte Katalysatorsuspension) enthält, in den Reaktor (1) mit Rührkesselcharakteristik (1) zurückführt.

2. Verfahren nach Anspruch 1, dadurch gekennzeichnet, daß der Reaktor (1) mit Rührkesselcharakteristik ein Mammutschlaufenreaktor ist.

3. Verfahren nach Anspruch 1 oder 2, dadurch gekennzeichnet, daß der Reaktor mit Rohrcharakteristik (2) eine Wirbelzellenkolonne ist.

4. Verfahren nach einem der Ansprüche 1 bis 3, dadurch gekennzeichnet, daß der Reaktor mit Rohrcharakteristik (2) in den Reaktor mit Rührkesselcharakteristik (1) integriert ist.

5. Verfahren nach einem der Ansprüche 1 bis 3, dadurch gekennzeichnet, daß der Einsatz dem Reaktor mit Rührkesselcharakteristik (1) durch das untere Ende des Reaktors mit Rohrcharakteristik (2) hindurch zugeführt wird.

6. Verfahren nach einem der Ansprüche 1 bis 3, dadurch gekennzeichnet, daß die Produktentnahme in einer in der unteren Verbindung zwischen den Reaktoren (1, 2) angeordneten Trennvorrichtung (13) vorgenommen wird.

7. Verfahren nach einem der Ansprüche 1 bis 6, dadurch gekennzeichnet, daß der Reaktor mit Rührkesselcharakteristik (1) über eine wenig unterhalb seines Gemischspiegels (10) angeordnete Umgehungsleitung (19) mit dem unteren Bereich des Reaktors mit Rohrcharakteristik (2) verbunden ist.

8. Verfahren nach einem der Ansprüche 1 bis 7, dadurch gekennzeichnet, daß der Reaktor mit Rührkesselcharakteristik (1) über eine wenig oberhalb seines Gemischspiegels (10) angeordnete Rückflußleitung (30) mit einem sich oberhalb der Einmündung der oberen Verbindungsleitung (3) in den Reaktor mit Rohrcharakteristik (2) befindenden Bereich dieses Reaktors (2) verbunden ist.

9. Verfahren nach Anspruch 8, dadurch gekennzeichnet, daß in der Rückflußleitung (30) eine Drosseleinrichtung, insbesondere ein Drosselventil (36) angeordnet ist.

10. Verfahren nach einem der Ansprüche 1 bis 9, dadurch gekennzeichnet, daß einem Reaktor mit Rührkesselcharakteristik (1) zwei oder mehrere Reaktoren mit Rohrcharakteristik (2) zugeordnet sind.

11. Verfahren nach einem der Ansprüche 1 bis 10, dadurch gekennzeichnet, daß zwei oder mehrere Reaktoren mit Rührkesselcharakteristik (1) mit je wenigstens einem zugeordneten Reaktor mit Rohrcharakteristik (2) hintereinandergeschaltet sind.

12. Verfahren nach einem der Ansprüche 1 bis 11, dadurch gekennzeichnet, daß in der unteren Verbindungsleitung (4) zwischen dem Reaktor mit Rührkesselcharakteristik (1) und dem Reaktor mit Rohrcharakteristik (2) eine Drossel- bzw. eine Zuteilungseinrichtung (38) angeordnet ist.

13. Verfahren nach einem der Ansprüche 7 bis 12, dadurch gekennzeichnet, daß in der Umgehungsleitung (19) eine in Richtung zum Reaktor mit Rohrcharakteristik (2) hin fördernde Pumpe (37) angeordnet ist.

## Claims

1. Method for carrying out reactions and interchange of substances in heterogeneous, fluid systems containing suspended solid matter (solid-fluid, solid-fluid-gaseous) by utilisation of an agitator-type reactor connected with a successive tube-type reactor thereby characterized that the lower end of the tube-type reactor (2) is connected with the lower end of the agitator-type reactor (1) and the product flow is taken from the tube-type reactor (2) and only part of the flow containing the solid matter of the reaction mixture (concentrated catalyst suspension) is returned into the agitator-type reactor (1).

2. Method according to claim 1 thereby characterized that the agitator-type reactor (1) is a gas lift loop reactor.

3. Method according to claim 1 and 2 thereby characterized that the tube-type reactor (2) is a vortex cell column.

4. Method according to one of the claims 1 to 3 thereby characterized that the tube-type reactor (2) is integrated into the agitator-type reactor (1).

5. Method according to one of the claims 1 to 3 thereby characterized that the product flow is fed into the agitator-type reactor (1) through the lower end of the tube-type reactor (2).

6. Method according to one of the claims 1 to 3 thereby characterized that the product removal is effected by a separating device (13) located in the lower connection between the reactors (1, 2).

7. Method according to one of the claims 1 to 6 thereby characterized that the agitator-type reactor (1) is connected with the lower zone of the tube-type reactor (2) via a by-pass (19) located just below its mixture level.

8. Method according to one of the claims 1 to 7 thereby characterized that the agitator-type reactor (1) is connected by a return (30) from just above its mixture level (10) to the tube-type reactor (2) above the junction of the upper connection line (3).

9. Method according to claim 8 thereby characterized that a throttle device, in particular a throttle valve (36) is located in the return line (30).

10. Method according to one of the claims 1 to 9 thereby characterized that two or more tube-type reactors (2) are allocated to one agitator-type reactor (1).

11. Method according one of the claims 1 to 10 thereby characterized that two or more agitator-type reactors (1) are each connected in series with at least one allocated tube-type reactor (2).

12. Method according to one of the claims 1 to 11 thereby characterized that a throttle and/or feeding device (38) is located in the lower connecting line (4) between the agitator-type reactor (1) and the tube-type reactor (2).

13. Method according to one of the claims 7 to 12 thereby characterized that a pump (37) feeding the tube-type reactor (2) is located in the by-pass (19).

**Revendications**

1. Procédé pour l'exécution des réactions et procédés d'échange de matière dans des systèmes hétérogènes et fluides et contenant des matières solides suspendues (solide-liquide, solide-liquide-gazeuse) en utilisant un réacteur du type agitateur, lié avec un réacteur du type tube qui suit, caractérisé par le fait que l'on lie le bout inférieur d'un réacteur type tube (2) avec le bout inférieur d'un réacteur type agitateur (1) et que l'on enlève le courant le produit du réacteur type tube (2) et retourne seulement une partie du courant contenant les constituants solides du mélange de reaction (suspension du catalyseur épaissie) dans le réacteur (1) type agitateur.

2. Procédé selon revendication 1, caractérisé par le fait que le réacteur (1) type agitateur est une colonne mammouth à bulles à écoulement en boucles.

3. Procédé selon revendication 1 ou 2, caractérisé par le fait que le réacteur type tube (2) est une colonne de cellule à tourbillon.

4. Procédé selon une des revendications 1 à 3, caractérisé par le fait que le réacteur type tube (2) est intégré dans le réacteur type agitateur (1).

5. Procédé selon une des revendications 1 à 3, caractérisé par le fait que la charge est alimentée au réacteur type agitateur (1) par le bout inférieur du réacteur type tube (2).

6. Procédé selon une des revendications 1 à 3, caractérisé par le fait que le prélèvement de produit est effectué dans un purgeur (13) disposé dans la jonction inférieure entre les réacteurs (1, 2).

7. Procédé selon une des revendications 1 à 6, caractérisé par le fait que le réacteur type agitateur (1) est lié avec la zone inférieure du réacteur type tube (2) à travers un by-pass (19) qui est disposé un peu en aval de son niveau de mélange (10).

8. Procédé selon une des revendications 1 à 7, caractérisé par le fait que le réacteur type agitateur (1) est lié à travers une conduite de retour (30) disposé un peut en amont de son niveau de mélange (10) avec la zone de ce réacteur (2) qui se trouve en amont de la débouchure de la tuyauterie supérieure de liaison (3) dans le réacteur type tube (2).

9. Procédé selon revendication 8, caractérisé par le fait qu'un dispositif d'etranglement, surtout une soupape d'etranglement (36) est disposé dans la conduite de retour.

10. Procédé selon une des revendications 1 à 9, caractérisé par le fait que deux ou plusieurs réacteurs type tube (2) sont attribués à un réacteur type agitateur (1).

11. Procédé selon une des revendications 1 à 10, caractérisé par le fait que deux ou plusieurs réacteurs type agitateur (1) avec chacun au moins un réacteur type tube attribué (2) sont disposés en série.

12. Procédé selon une des revendications 1 à 11, caractérisé par le fait qu'un étranglement et/ou un dispositif de dosage (38) est disposé dans la tuyauterie de liaison (4) entre le réacteur type agitateur (1) et le réacteur type tube (2).

13. Procédé selon une des revendications 7 à 12, caractérisé par le fait qu'une pompe (37) refoulant en direction du réacteur type tube (2) est disposée dans le by-pass (19).

Fig.1

Fig. 2

Fig. 3

Fig. 5

Fig. 4

Fig.6

Fig.7

Fig.8

Fig.9

EP 0 154 334 B1

Fig.10

Fig.11

Fig. 12

Fig. 13

EP 0 154 334 B1